(19) Europäisches Patentamt
European Patent Office
Office européen des brevets

(11) Veröffentlichungsnummer: **0 355 364 A2**

(12) # EUROPÄISCHE PATENTANMELDUNG

(21) Anmeldenummer: **89112609.6**

(22) Anmeldetag: **10.07.89**

(51) Int. Cl.⁴: **C07C 211/51 , A61K 7/13**

Die Bezeichnung der Erfindung wurde geändert (Richtlinien für die Prüfung im EPA, A-III, 7.3).

(30) Priorität: **18.07.88 DE 3824299**

(43) Veröffentlichungstag der Anmeldung: **28.02.90 Patentblatt 90/09**

(84) Benannte Vertragsstaaten: **ES GR**

(71) Anmelder: **Henkel Kommanditgesellschaft auf Aktien**
**Postfach 1100 Henkelstrasse 67**
**D-4000 Düsseldorf 1(DE)**

(72) Erfinder: **Rose, David, Dr.**
**Am Eichelkamp 223**
**D-4010 Hilden(DE)**
Erfinder: **Höffkes, Horst, Dr.**
**Carlo-Schmid-Strasse 113**
**D-4000 Düsseldorf(DE)**
Erfinder: **Lieske, Edgar**
**Hunsrückenstrasse 40**
**D-4000 Düsseldorf(DE)**

(54) **3,5-Diaminophenyl-alkylamine und deren Verwendung als Haarfärbemittel.**

(57) 3,5-Diaminophenyl-alkylamine der Formel

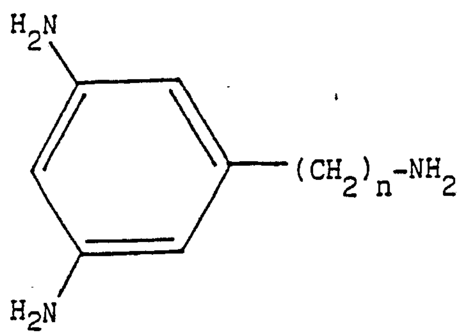

und deren Salze eignen sich als Kuppler zur Herstellung von Oxidationshaarfärbemitteln auf Basis üblicher Entwicklerverbindungen. Bevorzugt eignet sich das 3,5-Diaminophenyl-benzylamin als Kuppler für solche Oxidationshaarfärbemittel, die als Entwicklersubstanz 2,4,5,6-Tetraaminopyrimidin oder dessen Derivate enthalten, zur Ausbildung roter bis brauner Nuancen mit guten Echtheitseigenschaften.

EP 0 355 364 A2

## Haarfärbemittel

Gegenstand der Erfindung sind Haarfärbemittel auf der Basis von Oxidationsfarbstoffen. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt, die unter dem Einfluß von Oxidationsmitteln oder von Luftsauerstoff Farbstoffe ausbilden. Als kosmetische Träger für die Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haaren spielen die sogenannten Oxidationsfarben, die durch oxidative Kupplung einer oder mehrerer Entwicklerkomponenten untereinander oder mit einer oder mehreren Kupplerkomponenten entstehen, wegen ihrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate, 4-Aminopyrazolonderivate und Tetraaminopyrimidine eingesetzt. Als sogenannte Kupplersubstanzen werden m-Phenylendiaminderivate, Naphthole, Resorcinderivate und Pyrazolone verwendet.

Gute Oxidationshaarfarbstoffvorprodukte müssen in erster Linie folgende Voraussetzungen erfüllen: Sie müssen bei der oxidativen Kupplung die gewünschten Farbnuancen in ausreichender Intensi tät ausbilden. Sie müssen ferner ein gutes Aufziehvermögen auf menschlichem Haar besitzen, ohne die Kopfhaut zu stark anzufärben. Auch soll der Farbaufzug gleichmäßig erfolgen, d.h. die stärker strapazierten Haarspitzen sollen nicht stärker gefärbt werden als der wenig geschädigte Haaransatz. Die damit erzeugten Färbungen müssen eine hohe Stabilität gegen Wärme, Licht und die bei der Dauerwellung des Haars verwendeten Chemikalien aufweisen. Schließlich sollen die Oxidationshaarfarbstoffvorprodukte in toxikologischer und dermatologischer Hinsicht unbedenklich sein.

3,5-Diamino-phenylalkanole sind als Oxidationsfarbstoffvorprodukte z.B. aus DE 35 08 309 A1 bekannt. Auch 3,5-Dihydroxyphenyl-alkylamine eignen sich, z.B. gemäß DE 32 33 541 A1 als Kuppler für Oxidationshaarfärbemittel. Die aus den genannten Druckschriften bekannten Kuppler haben auch die Eigenschaft, mit Entwicklern vom Typ des 2,4,5,6-Tetraaminopyrimidins rote oder braune Nuancen auszubilden. Diese haben jedoch den Nachteil, daß sie ungleichmäßig auf das Haar aufziehen und z.B. die stärker strapazierten Haarspitzen wesentlich stärker anfärben als die weniger geschädigten Bereiche des Haaransatzes.

Es wurden nun neue Stoffe gefunden, die sich als Kuppler für Oxidationshaarfärbemittel und insbesondere als Rotkuppler für Entwickler des Typs 2,4,5,6-Tetraaminopyrimidin gut eignen, aber die obengenannten Nachteile nicht aufweisen.

Gegenstand der Erfindung sind 3,5-Diaminophenyl-alkylamine der Formel (I)

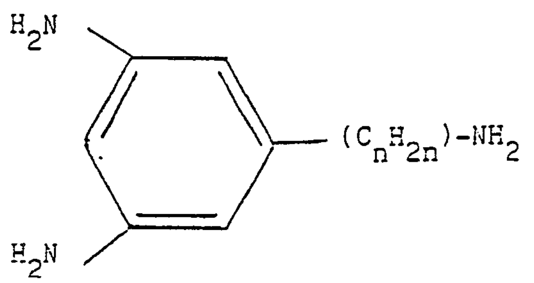

(I)

in der n = 1 oder 2 ist, oder deren Salze. Diese Verbindungen sind neu. Sie lassen sich leicht nach ansich literaturbekannten Verfahren herstellen:

2,4-Diamino-benzylamin kann man z.B. durch katalytische Hydrierung aus 3,5-Dinitro-benzonitril nach der in Beispiel 1.1 angegebenen Arbeitsweise herstellen. Eine andere Möglichkeit besteht in der Umsetzung von 3,5-Diaminophenyl-alkylhalogeniden oder 3,5 Diaminophenyl-alkanolen oder deren p-Toluolsulfonsäureestern mit Ammoniak.

Die 3,5-Diamonophenyl-alkylamine der Formel (I) sind als Kupplersubstanzen für eine Vielzahl bekannter Entwicklerverbindungen geeignet und erzeugen besonders brillante Färbungen mit hoher Licht- und Wärmestabilität. Als Entwicklersubstanzen können in den erfindungsgemäßen Haarfärbemitteln z.B. aromatische Amine mit einer oder mehreren weiteren $NH_2$-Gruppen, NHR-Gruppen, $NR_2$-Gruppen, wobei R eine Alkylgruppe mit 1-4 C-Atomen oder eine Hydroxyalkylgruppe oder eine Aminoalkylgruppe mit 2-4 C-Atomen darstellt, Aminophenole, Aminophenolether, Diaminopyridinderivate oder 2,4,5,6-Tetraaminopyrimidin und dessen Derivate eingesetzt werden.

Bevorzugt wegen seiner guten Zugänglichkeit wird das 3,5-Diamino-benzylamin eingesetzt. 3,5 Diaminophenyl-alkylamine der Formel (I) stellen besonders wertvolle Kupplerkomponenten für Entwickler vom Typ des 2,4,5,6-Tetraaminopyrimidins und dessen Derivate dar, da sie mit diesen Entwicklerkomponenten rote oder braunrote Nuancen ausbilden, die ein besonders gleichmäßiges Aufziehvermögen auf das Haar im Bereich des Haaransatzes und der Haarspitzen, d.h. ein besonders gutes Egalisiervermögen, aufweisen. Ein Vergleich des Egalisiervermögens eines erfindungsgemäßen Haarfärbemittels mit 3,5-Diaminophenyl-benzylamin als Kuppler und 2,4,5,6-Tetraaminopyrimidin als Entwickler mit dem eines sonst gleichen Haarfärbemittels mit dem als Rotkuppler für 2,4,5,6-Tetraaminopyrimidin bekannten 2-Methylresorcin zeigt, daß mit dem erfindungsgemäßen Haarfärbemittel die Farbdifferenz zwischen einer angefärbten, geschädigten Haarsträhne und einer angefärbten, ungeschädigten Haarsträhne auf die Hälfte des mit dem herkömmlichen Färbemittel mit 2-Methylresorcin als Kuppler erzielbaren Wertes verringert wird.

Ein besonders bevorzugter Erfindungsgegenstand sind daher erfindungsgemäße Haarfärbemittel, die als Entwicklersubstanz 2,4,5,6-Tetraaminopyrimidin oder dessen Derivate mit der Formel (II)

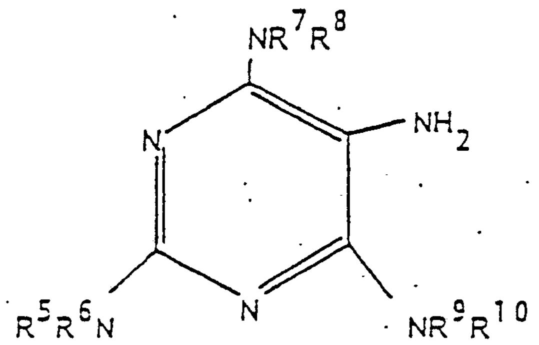

(II)

in der $R^5$-$R^{10}$ unabhängig voneinander Wasserstoff, Alkylgruppen mit 1-4 C-Atomen oder Hydroxyalkylgruppen mit 2-4 C-Atomen oder die Gruppen $R^5$ und $R^6$ bzw. $R^7$ und $R^8$ bzw. $R^9$ und $R^{10}$ jeweils gemeinsam mit dem Stickstoffatom einen Morpholin-, Piperidin-, Pyrrolidin- oder Piperazinring bilden oder deren Salze enthalten sind. Bevorzugt wird als Entwicklerkomponente der Formel (II) das 2,4,5,6-Tetraaminopyrimidin verwendet.

In die erfindungsgemäßen Haarfärbemittel können die 3,5-Diaminophenyl-alkylamine der Formel I sowie die Tetraaminopyrimidine der Formel (II) entweder in freier Form oder auch in Form von Salzen der Aminogruppen mit anorganischen oder organischen Säuren, z.B. in Form der Hydrochloride, Sulfate, Phosphate, Acetate, Propionate, Lactate oder Citrate, eingesetzt werden.

Die erfindungsgemäßen Haarfärbemittel können neben den 3,5-Diamino-phenyl-alkylaminen der allgemeinen Formel (I) auch andere bekannte Kupplersubstanzen enthalten, die zur Modifizierung der Farbnuancen und zur Erzeugung natürlicher Farbtöne erforderlich sind. Solche üblichen Kupplerverbindungen sind z.B. andere m-Phenylendiamine, z.B. 2,4-Diaminophenyl-2-hydroxyethylether, oder Phenole, Resorcine, m-Aminophenole, Naphthole oder Pyrazolone.

Zur Erzeugung natürlicher blonder, brauner oder schwarzer Haarfärbungen mit einem Oxidationshaarfärbemittel mit einem Entwickler vom Typ der 2,4,5,6-Tetraaminopyrimidine der Formel (II) ist es z.B. erforderlich, zusätzlich zu den Rotkupplern vom Typ der 3,5-Diaminophenyl-alkylamine der Formel I noch Gelbkuppler und/oder Blaukuppler für diesen Entwickler einzusetzen, die ebenfalls mit Tetraaminopyrimidinen gut aufziehende Farbstoffe mit hohem Egalisierungsvermögen ausbilden. Besonders geeignete Gelbkuppler sind z.B. 2,7-Dihydroxynaphthalin und 2,6-Dihydroxy-3,4-dimethylpyridin. Ein besonders geeigneter Blaukuppler ist z.B. 2-Methyl-5-amino-6-chlorphenol.

Eine besonders bevorzugte Ausführungsform der Erfindung ist daher ein erfindungsgemäßes Haarfärbemittel mit einem 2,4,5,6-Tetraaminopyrimidin als Entwicklersubstanz, welches neben dem 3,5-Diaminophenyl-alkylamine der Formel (I) als weitere Kuppler einen oder mehrere Kuppler aus der Gruppe 2,7-Dehydroxynaphthalin, 2 ,6-Dihydroxy-3 ,4-dimethylpyridin und/oder 2-Methyl-5-amino-6-chlorphenol enthält.

Gegebenenfalls können auch direktziehende Farbstoffe zusätzlich zur weiteren Modifizierung der Farbnuancen eingesetzt werden. Solche direktziehenden Farbstoffe sind z.B. Nitrophenylendiamine, Nitroaminophenole, Anthrachinonfarbstoffe oder Indophenole. Zu den erfindungsgemäßen Haarfärbemitteln werden die 3,5-Diamino phenyl-alkylamine der Formel (I) und die gegebenenfalls zusätzlich vorhandenen bekannten Kupplersubstanzen im allgmeinen in etwa molaren Mengen, bezogen auf die verwendeten

Entwicklersubstanzen, eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklersubstanzen und Kupplersubstanzen in einem Molverhältnis von 1 : 0,5 bis 1 : 2 enthalten sein können.

Es ist nicht erforderlich, daß die 3,5-Diaminophenyl-alkylamine der Formel (I) sowie die sonst in den Haarfärbemitteln vorhandenen Oxidationsfarbstoffvorprodukte oder direkt ziehenden Farbstoffe einheitliche chemische Verbindungen darstellen. Vielmehr können diese auch Gemische der erfindungsgemäß einzusetzenden Kuppler- oder Entwicklersubstanzen sein.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Zur Herstellung der erfindungsgemäßen Haarfärbemittel werden die Oxidationsfarbstoffvorprodukte in einen geeigneten kosmetischen Träger eingearbeitet. Solche Träger sind z.B. Cremes, Emulsionen, Gele oder auch tensidhaltige, schäumende Lösungen, z.B. Shampoos oder andere Zubereitungen, die für die Anwendung auf dem Haar geeignet sind. Übliche Bestandteile solcher kosmetischer Zubereitungen sind z.B. Netz- und Emulgiermittel wie anionische, nichtionische oder ampholytische Tenside, z.B. Seifen, Fettalkoholsulfate, Alkansulfonate, alpha-Olefinsulfonate, Fettalkoholpolyglykolethersulfate, Ethylenoxidanlagerungsprodukte an Fettalkohole, an Fettsäuren und an Alkylphenole, Sorbitanfettsäureester und Fettsäurepartialglyceride, Fettsäurealkanolamide sowie Verdickungsmittel wie z.B. Methyl- oder Hydroxyethylcellulose, Stärke, Fettkomponenten wie z.B. Fettalkohole, Paraffinöle oder Fettsäureester, ferner Parfümöle und haarpflegende Zusätze, wie z.B. wasserlösliche kationische Polymere, Proteinderivate, Pantothensäure und Cholesterin.

Die Bestandteile der kosmetischen Träger werden zur Herstellung der erfindungsgemäßen Haarfärbemittel in für diese Zwecke üblichen Mengen eingesetzt, z.B. werden Emulgiermittel in Konzentrationen von 0,5 - 30 Gew.-% und Verdickungsmittel in Konzentrationen von 0,1 - 25 Gew.-% des gesamten Färbemittels eingesetzt.

Besonders geeignet als Träger ist eine Öl-in-Wasser-Emulsion mit einem Gehalt von 0,1 - 25 Gew.-% einer Fettkomponente und 0,5 -30 Gew.-% eines Emulgiermittels aus der Gruppe der anionischen, nichtionischen oder ampholytischen Tenside.

Die Oxidationsfarbstoffvorprodukte werden in Mengen von 0,2 - 5 Gew.-%, vorzugsweise 1 - 3 Gew.-% des gesamten Färbemittels in den Träger eingemischt. Der Gehalt an 3,5-Diaminophenyl-alkylaminen der Formel (I) kann in den erfindungsgemäßen Haarfärbemitteln etwa 0,05 - 10 Millimol pro 100 g des Haarfärbemittels betragen.

Die Anwendung der erfindungsgemäßen Haarfärbemittel kann, unabhängig von der Art der kosmetischen Zubereitung, z.B. als Creme, Gel oder Shampoo, bevorzugt in einem pH-Bereich von 6 -10 erfolgen. Besonders bevorzugt ist die Anwendung der Haarfärbemittel in einem schwach alkalischen Milieu. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo verwendet wurde.

Die nachfolgenden Beispiele sollen den Erfindungsgegenstand näher erläutern ohne ihn jedoch hierauf zu beschränken.

## Beispiele

### 1. Herstellungsbeispiele

#### 1.1 Herstellung von 3,5-Diaminobenzylamin-trihydrochlorid

8 g (41,5 mMol) 3,5-Dinitrobenzoesäurenitril wurden in 120 ml Essigsäureanhydrid gelöst und in Gegenwart von Raney-Niekel bei 50 °C und 50 bar Wasserstoffdruck hydriert. Nach Beendigung der Wasserstoff-Aufnahme wurde der Katalysator durch Filtration entfernt und die Lösung zur Trockene

eingeengt. Der Rückstand wurde mit 100 ml 50 %iger Salzsäure 5 Stunden unter Rückfluß zum Sieden erhitzt und dann zur Trockene eingeengt. Es wurden braune Kristalle mit einem Schmelzpunkt ab 220 °C (unter Zersetzung) in einer Ausbeute von 9,2 g (37,4 mMol, ca. 90 % d.Th) erhalten.

2. Anwendungsbeispiele

Es wurden erfindungsgemäße Haarfärbemittel in Form einer Haarfärbe-Cremeemulsion der folgenden Zusammensetzung hergestellt:

| | |
|---|---|
| Fettalkohol $C_{12}$-$C_{18}$ | 10 g |
| Fettalkohol $C_{12}$-$C_{14}$ + 2EO-sulfat Na-Salz, 28 %ig | 25 g |
| Wasser | 60 g |
| 1. Entwickler (Komponente E) | 7,5 mMol |
| Kupplerkomponente | 7,5 mMol |
| $Na_2SO_3$ (Inhibitor) | 1,0 g |
| konzentrierte Amoniak-Lösung | bis pH = 9,5 |
| Wasser | ad 100 g |

Die Bestandteile wurden der Reihe nach miteinander vermischt. Nach Zugabe der Oxidationsfärbemittelvorprodukte und des Inhibitors wurde zunächst mit konzentrierter Amoniak-Lösung der pH-Wert der Emulsion auf 9,5 eingestellt, dann wurde mit Wasser auf 100 g aufgefüllt.

Die oxidative Entwicklung der Färbung wurde mit 3 %iger Wasserstoffperoxidlösung als Oxidationsmittel durchgeführt. Hierzu wurden 100 g der Emulsion mit 50 g Wasserstoffperoxidlösung (3 %ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Als Kupplerkomponente wurde die Verbindung gemäß Beispiel 1.1
3,5-Diaminobenzylamin-trihydrochlorid
eingesetzt.

Als Entwicklerkomponenten wurden die in der folgenden Tabelle angegebenen Verbindungen eingesetzt. Die Nuancen der auf diese Weise entstandenen Haaranfärbungen sind der Tabelle zu entnehmen.

Tabelle

| Nr. | Entwickler (Komponente E) | Nuance des gefärbten Haares |
|---|---|---|
| 2.1 | 2,4,5,6,-Tetraaminopyrimidin | granatrot |
| 2.2 | 2,5,6-Triamino-4-hydroxypyrimidin | violettbraun |
| 2.3 | 2-Dimethylamino-4,5,6-triaminopyrimidin | dunkelmagenta |
| 2.4 | 2-Methylamino-4,5,6-triamino-pyrimidin | dunkelmagenta |
| 2.5 | 2-Morpholino-4,5,6-triaminopyrimidin | dunkelmagenta |
| 2.6 | 2-Piperidino-4,5,6-triaminopyrimidin | dunkelpurpur |
| 2.7 | p-Aminophenol | violettgrau |
| 2.8 | p-Toluylendiamin | blauschwarz |
| 2.9 | N,N-Diethyl-p-phenylendiamin | dunkelblau |
| 2.10 | N-(2-hydroxyethyl)-p-phenylendiamin | schwarzblau |
| 2.11 | N,N-Bis-(2-hydroxyethyl)-p-phenylendiamin | dunkelblau |
| 2.12 | 2-chlor-p-phenylendiamin | dunkelviolett |
| 2.13 | (2,5-Diamonophenyl)-2-ethoxy-ethyl-ether | purpurgrau |
| 2.14 | 1,2-Bis-(4-aminophenyl)-ethylendiamin | violettgrau |
| 2.15 | 2,5-Diaminobenzylalkohol | schwarzblau |
| 2.16 | 2-(2,5-Diaminophenyl)-ethanol | schwarzblau |

## Ansprüche

1. 3,5-Diaminophenyl-alkylamine der Formel (I)

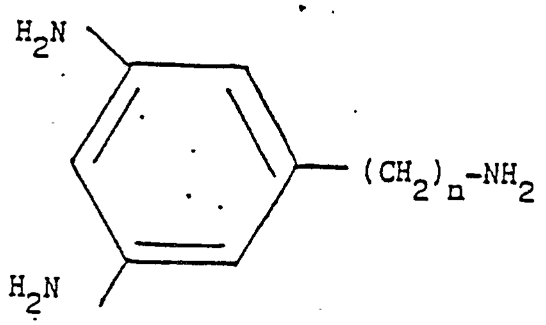

(I)

in der n = 1 oder 2 ist, oder deren Salze.

2. Haarfärbemittel, enthaltend Oxidationsfarbstoffvorprodukte in einem Träger, dadurch gekennzeichnet, daß als Oxidationsfarbstoffvorprodukte 3,5-Diaminophenyl-alkylamine der Formel (I)

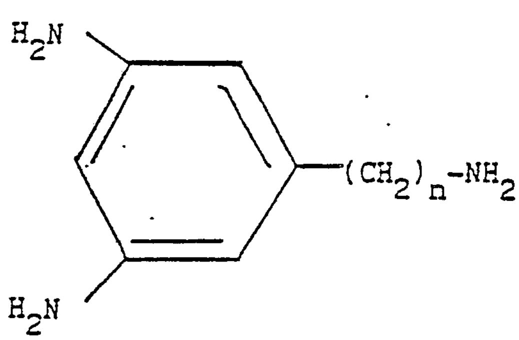

(I)

in der n = 1 oder 2 ist, oder deren Salze, als Kupplersubstanzen und die in Oxidationshaarfärbemitteln üblichen Entwicklersubstanzen enthalten sind.

3. Haarfärbemittel nach Anspruch 2, dadurch gekennzeichnet, daß als Oxidationsfarbstoffvorprodukt 3,5-Diaminobenzylamin oder dessen Salze als Kupplersubstanzen enthalten sind.

4. Haarfärbemittel nach einem der Ansprüche 2 oder 3, dadurch gekennzeichnet, daß als Entwicklersubstanz 2,4,5,6-Tetraaminopyrimidin oder dessen Derivate mit der Formel II

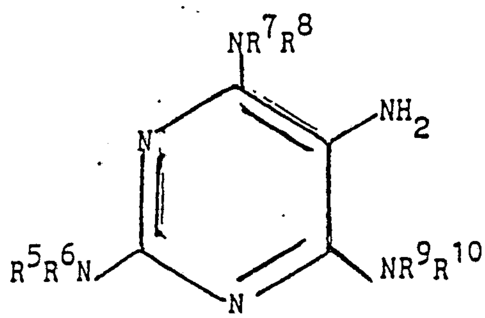

(II)

in der $R^5$ bis $R^{10}$ unabhängig voneinander Wasserstoff, Alkylgruppen mit 1-4 C-Atomen oder Hydroxyalkylgruppen mit 2-4 C-Atomen oder die Gruppen $R^5$ und $R^6$ bzw. $R^7$ und $R^8$ bzw. $R^9$ und $R^{10}$ jeweils gemeinsam mit dem Stickstoffatom einen Morpholin-, Piperidin-, Pyrrolidin- oder Piperazinring bilden oder deren Salze enthalten sind.

5. Haarfärbemittel nach Anspruch 4, dadurch gekennzeichnet, daß zusätzlich einer oder mehrere Kuppler aus der Gruppe 2,7-Dihydroxynaphthalin, 2,6-Dihydroxy-3,4-dimethylpyridin und/oder 2-Methyl-5-amino-6-chlorphenol enthalten sind.

6. Haarfärbemittel nach den Ansprüchen 2 bis 5, dadurch gekennzeichnet, daß als Träger eine Öl-in-Wasser-Emulsion mit einem Gehalt von 0,1 bis 25 Gew.-% einer Fettkomponente und 0,5 bis 30 Gew.-% eines Emulgiermittels aus der Gruppe der anionischen, nichtionischen oder ampholytischen Tenside und als Oxidationsfarbstoffvorprodukte 3,5-Diaminophenylalkylamine der Formel (I) in einer Menge von 0,05 bis 10 Millimol pro 100 g des Haarfärbemittels enthalten sind.